(19) Europäisches Patentamt
European Patent Office
Office européen des brevets

(11) Publication number: **0 024 761**
**A1**

(12) # EUROPEAN PATENT APPLICATION

(21) Application number: 80200774.0

(22) Date of filing: 18.08.80

(51) Int. Cl.[3]: C 07 C 27/22
C 07 C 45/50, C 07 C 29/16
B 01 J 31/20, B 01 J 31/24

(30) Priority: 29.08.79 GB 7929955

(43) Date of publication of application:
11.03.81 Bulletin 81/10

(84) Designated Contracting States:
DE FR GB IT NL

(71) Applicant: SHELL INTERNATIONALE RESEARCH MAATSCHAPPIJ B.V.
Carel van Bylandtlaan 30
NL-2596 HR DEN HAAG(NL)

(72) Inventor: Janssen, Johannes Jozefus
Carel van Bylandtlaan 30
NL-2596 HR The Hague(NL)

(74) Representative: Keuzenkamp, Abraham et al,
c/o Shell Internationale Research Maatschappij B.V. P.O. Box 302
NL-2501 CH 's-Gravenhage(NL)

(54) **Process for the preparation of alcohols or aldehydes, alcohols or aldehydes prepared by this process and stabilized compositions suitable for use in the said process.**

(57) In the hydroformylation of a compound having a carbon-carbon double bond with formation of alcohols and/or aldehydes in the presence of a transition metal (e.g. cobalt) carbonyl phosphine (or arsine, stibine) complex catalyst, deposition of transition metal on walls is considerably reduced and the catalyst is rendered considerably more stable by the presence of a dissolved salt of an alkyl-substituted arylsulphonic acid salt.

EP 0 024 761 A1

# PROCESS FOR THE PREPARATION OF ALCOHOLS OR ALDEHYDES, ALCOHOLS OR ALDEHYDES PREPARED BY THIS PROCESS AND STABILIZED COMPOSITIONS SUITABLE FOR USE IN THE SAID PROCESS

The invention relates to a process for the preparation of alcohols and/or aldehydes by catalytic hydroformylation. The invention also relates to stabilized compositions containing a hydroformylation catalyst.

Hydroformylation is well known in the art and comprises converting a compound having a carbon-carbon double bond to a corresponding primary alcohol and/or aldehyde, in which the hydroxymethyl or formyl group is bound to one of the carbon atoms previously involved in the double bond. The carbon-carbon double bond is simultaneously saturated with the addition of hydrogen and carbon monoxide to form the alcohol or aldehyde. Thus, hydroformylation may, in the general case, be represented by the following reaction schemes:

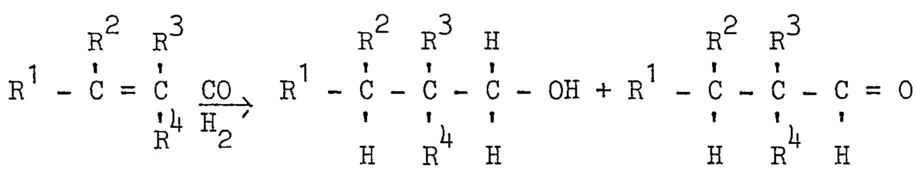

(1)

and

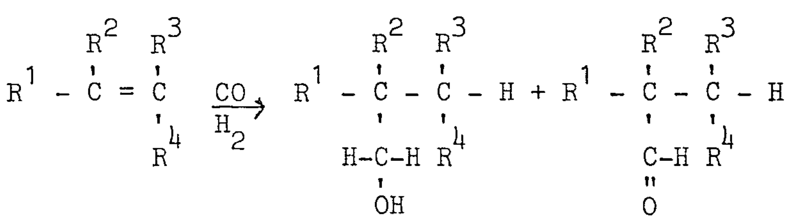

(2)

In the above schemes each R represents an organic radical or a suitable atom such as a hydrogen or halogen atom. The above reactions are applicable to aliphatic carbon-carbon

double bonds, both acyclic and alicyclic. In the latter case $R^2$ and $R^3$ are joined into a divalent radical such as tetramethylene. Shifting of the original double bond may lead to different compounds having a carbon-carbon double bond, in which case the alcohol or aldehyde is varied accordingly.

Dicobalt octa-carbonyl was widely and still is used as hydroformylation catalyst. This catalyst usually decomposes rapidly unless high pressures, say from 7-30 MPa, of carbon monoxide are maintained. Correspondingly high pressures of hydrogen are also necessary for the desired hydroformylation. Moreover, this catalyst favours reaction scheme (2), leading to undesired branched primary alcohols and branched aldehydes. Improved hydroformylation catalysts which comprise certain transition metal complexes with bi-phyllic ligands containing trivalent phosphorus, trivalent arsenic or trivalent antimony have been described in British patent specifications 988,941, 1,109,787, 1,110,549, 1,191,815, 1,127,965 and 1,254,063. These catalysts have considerable advantages over the former dicobalt octa-carbonyl, in particular because of their increased stability, as a result of which they allow the hydroformylation reaction to proceed at much lower pressures and because reaction scheme (1) is favoured, leading to formation of less branched alcohols and aldehydes. Moreover, the aldehydes are simultaneously hydrogenated to the corresponding alcohols.

A hydroformylation can be conducted continuously by contacting a compound having a carbon-carbon double bond with a gaseous mixture containing carbon monoxide and hydrogen in the presence of the said improved hydroformylation catalysts, for example at a temperature in the range of from 125 to 210°C and a pressure in the range of from 3 to 9 MPa. The reaction mixture obtained can be separated by flashing at sub-atmospheric pressure into an alcohol-containing distillate and a residue containing dissolved hydroformylation catalyst and dissolved

catalytically inactive transition metal compounds. Reacting this residue with carbon monoxide, for example by recycling residue to the hydroformylation zone, results in conversion of dissolved catalytically inactive transition metal compounds into dissolved hydroformylation catalyst.

The above-mentioned improved hydroformylation catalysts have the disadvantage that under plant-operating conditions deposition of transition metal onto the inner walls of the hydroformylation vessel, of the flashing apparatus following the hydroformylation vessel and of appertaining pipelines may be observed. From time to time the deposited transition metal must be removed, which shortens the valuable on-stream time. Moreover, this removal must be carried out with corrosive liquids, for example with aqueous nitric acid, the handling of which needs a great deal of precautions. Simultaneously, a decrease in the contents of dissolved hydroformylation catalyst and dissolved catalytically inactive transition metal compounds in the liquid in the hydroformylation vessel is observed, which means a waste of valuable catalyst. Moreover, recycling of the said residue to the hydroformylation zone results in conversion of only a part of the dissolved catalytically inactive transition metal compounds into dissolved hydroformylation catalyst.

It has now been found that the presence of certain dissolved salts to be named hereinafter considerably reduces and often prevents the said deposition of transition metal, the inconvenient removal of deposited transition metal and the loss of on-stream time thus often being obviated; simultaneously, both the contents of the dissolved hydroformylation catalyst and the dissolved catalytically inactive transition metal compounds in the liquid in the hydroformylation vessel are increased; moreover, reacting the above-mentioned residue with carbon monoxide results in conversion of all of the dissolved catalytically inactive transition metal compounds into dissolved hydroformylation catalyst.

Accordingly, the invention provides a process for the preparation of alcohols and/or aldehydes, characterized in that a compound having a carbon-carbon double bond is reacted with carbon monoxide and hydrogen in the presence of (1) one or more complexes comprising in the molecule an atom of a transition metal having an atomic number of from 22 to 29, of from 40 to 47 or of from 72 to 79, carbon monoxide as a bi-phyllic ligand and at least one molecule of a bi-phyllic ligand containing trivalent phosphorus, trivalent arsenic or trivalent antimony, and (2) one or more dissolved salts of one or more alkyl-substituted arylsulphonic acids.

As used herein, the word "complex" indicates a co-ordination compound which is a combination of a transition-metal atom with one or more electron-rich molecules capable of independent existence. Such molecules, the number of which is ordinarily equal to the co-ordination number of the metal involved, are frequently indicated herein by the expression "biphyllic ligand", defined by R.G. Pearson, et al. in J. Am. Chem. Soc. 82, 787 (1960). The carbon monoxide molecule is an example of a biphyllic ligand and serves as such in the complexes. However, these catalysts must also contain a trivalent atom-containing ligand as mentioned above.

While various salts of alkyl-substituted arylsulphonic acids may be employed for the purpose of the present invention, the determining factor is that the salt of the particular alkyl-substituted arylsulphonic acid be soluble in the reaction medium. The aryl nucleus in the salts of the alkyl-substituted arylsulphonic acids is suitably derived from benzene, naphthalene or anthracene, and preferably from benzene. The alkyl group is preferably unsubstituted, but may be substituted with one or more atoms or groups as long as they do not unfavourably affect the hydroformylation reaction. One to five alkyl groups may be attached to the aryl nucleus and preferably one alkyl group is attached to it. The alkyl group or groups suitably have from

6 to 30 carbon atoms. The alkyl group or alkyl groups may be branched but preferably have a straight chain. Very good results have been obtained with mixtures of salts of alkyl-substituted arylsulphonic acids in which the alkyl groups are unbranched and are for the greater part bound with a tertiary carbon atom to the aryl group.. Examples of such unbranched alkyl groups are 1-methylpentyl, 1-ethylpentyl, 1-propylpentyl, 1-propylhexyl, 1-propylheptyl and 1-butylhexyl groups.

The salts in question are suitably alkali metal, alkaline earth metal or ammonium salts, i.e. salts of lithium, sodium, potassium, rubidium, cesium, magnesium, calcium, strontium or barium. Sodium salts are preferred. Very good results have been obtained with mixtures of sodium salts of p-alkylbenzenesulphonic acids, in which mixtures the alkyl groups have in the range of from 8 to 13 carbon atoms, are unbranched and are for the greater part bound with a tertiary carbon atom to the aromatic nucleus; below such mixtures in which the alkyl groups have of from 8 to 13 and of from 9 to 13 carbon atoms are also indicated by "SABS 813" and "SABS 913", respectively. Other examples of the salts in question are those derived from primary, secondary or tertiary amines or from tetra-alkylammonium hydroxides.

The salts of the alkyl-substituted arylsulphonic acids may be introduced into the reaction mixture as such or dissolved in a suitable solvent, for example in water or an alkanol, for example ethanol, 2-propanol, 1-butanol, 2-methyl-1-propanol or a n-alkanol having 7 to 15 carbon atoms per molecule (for example a mixture of primary alkanols having 7 to 9 or 9 to 11 carbon atoms per molecule, of which at least 80% has a straight carbon chain), or a mixture of these solvents. The salts are suitably dissolved in the alcohol being prepared.

Molar ratios of the salt of alkyl-substituted arylsulphonic acid to transition metal are not critical and are suitably in the range of from 0.005 to 2, particularly of from 0.1 to 1.

The biphyllic ligands may be monodentate or polydentate. Polydentate means that they contain more than one phosphorus, arsenic or antimony atom.

An example of the complexes which may be used according to the present invention are those having the general formula:

$$\left[(R^1{}_zA)_x - L - R_y\right]_p M(CO)_n$$

wherein $(R^1{}_zA)_x - L - R_y$ represents the biphyllic ligand, M is a transition metal having an atomic number of from 22 to 29, of from 40 to 47 or of from 72 to 79, L is phosphorus, arsenic or antimony, R and $R^1$ are alkyl, aryl, aralkyl, or alkaryl groups, A is oxygen, nitrogen, or sulphur, x ranges from 0 to 3, y = 3-x, p ranges from 1 to the co-ordination number of the transition metal, n + p is equal to said co-ordination number and z is 1 where A is oxygen or sulphur or is 2 when A is nitrogen. Such complexes are described in British patent specification 988,941; the biphyllic ligand may be a trialkylphosphine, for example a tertiary phosphine containing two methyl groups and an aliphatic group with 8 to 30 carbon atoms, for example dimethyldodecylphosphine.

Examples of biphyllic ligands which may be used in the process according to the present invention in combination with cobalt as the transition metal are:

(1) Heterocyclic phosphines, in which a heterocyclic group with at least five carbon atoms is present (described in British patent specification 1,109,787). Tertiary heterocyclic phosphines of this category, among which the tertiary 6-membered mono-heterocyclic phosphines, are examples of such ligands. Other examples are tertiary 7-membered mono-heterocyclic phosphines, such as 1-phenylphosphepane. In particular bicyclic heterocyclic tertiary phosphines have been found especially suitable; preferred among the latter are hydrocarbyl-substituted mono-phosphabicyclo hydrocarbons having

8 or 9 ring atoms including the phosphorus atom in which the smallest phosphorus-containing ring contains at least 5 atoms and the phosphorus atom is not a bridgehead atom. Among the latter phosphines 9-eicosyl-9-phosphabicyclo-[4.2.1] nonane and 9-eicosyl-9-phosphabicyclo [3.3.1] - nonane are preferred. Other examples of suitable phosphines are hydrocarbyl-substituted monophosphabicyclic nona-trienes.

(2) Tertiary five-membered heterocyclic phosphines, one of the members of the heterocyclic structure being a phosphorus atom (described in British patent specification 1,110,549). Complexes formed with such phosphines may have the general formula:

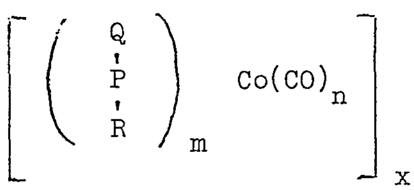

wherein Q represents 1,4-hydrocarbylene, R represents hydrocarbyl, m and n are integers, each having a value of at least 1 and whose sum is 4, and x is an integer from 1 to 3. Such complexes may, for example, contain a 1-hydrocarbyl-3-phospholine.

(3) Secondary phosphines in which the phosphorus atom is linked to a non-acetylenic hydrocarbyl group and to a perhydropentalenyl group (described in British patent specification 1,191,815). Examples of such phosphines are secondary hydrocarbyl-(octahydropentalenyl-1)phosphines, such as eicosyl(octahydropentalenyl-1)phosphine. The catalyst thus formed may be represented by the formula:

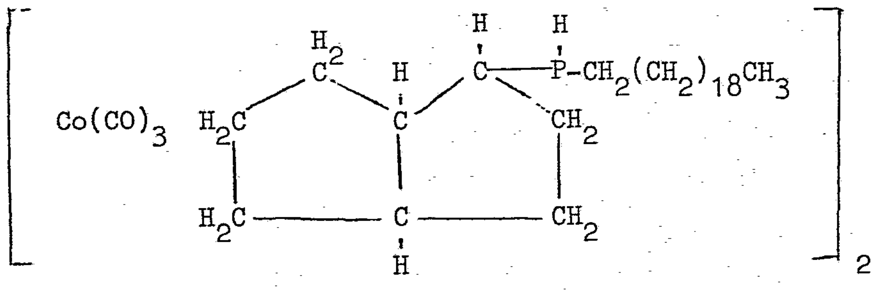

(4) Hydrocarbylene-bis(monophosphabicyclo hydrocarbons), in which each bicyclic structure the phosphorus atom - being linked to the hydrocarbylene group - is a member of a ring without being a bridgehead atom, and the smallest phosphorus-containing ring consists of at least five atoms (described in British patent specification 1,127,965). The bicyclic structures may be monophosphabicyclononanes; in the latter structures the hydrocarbylene group may be octamethylene and the monophosphabicyclononanes may be 9-phosphabicyclo [4.2.1] nonane or 9-phosphabicyclo [3.3.1] nonane.

(5) Bicyclic heterocyclic secondary phosphines in which a heterocyclic phosphorus-containing ring with at least five carbon atoms is present (described in British patent specification 1,254,063). Examples of such phosphines are 9-phosphabicyclo [3.3.1] nonane and 9-phosphabicyclo-[4.2.1] nonane.

It is within the scope of the invention to synthesize the catalyst in situ by reacting a suitable compound of the selected metal with the desired ligand.

The transition metals, atomic numbers and the groups mentioned in the present specification refer to the Periodic Table as shown on the inside of the cover of "Handbook of Chemistry and Physics", 59th Edition (1978) (Chem. Rubber Publ. Co.). It is preferred that the transition metal be one from Group VIII, cobalt being a particularly preferred metal. When the complex serving as catalyst is in its active form,

the transition metal will be in a reduced valence state. This will normally be zero and may be even lower, e.g. -1.

The stability of the hydroformylation catalyst used in the process of the present invention is further improved by carrying out this process in an alkaline medium.

Examples of bases useful for obtaining an alkaline medium are found in British patent specification 1,002,429. Inorganic basic compounds such as alkali and alkaline earth metal oxides and hydroxides, such as potassium hydroxide in particular, and salts of such hydroxides and weak acids (i.e. acids only partially ionized in dilute aqueous solutions), as well as various organic compounds having base groupings, as primary, secondary, or tertiary amines are particularly useful. Examples of primary amines are those having alkyl groups with 1 to 30 carbon atoms, for example a tertiary alkyl group of 12 to 18 carbon atoms.

Ratios of catalyst to compound having a carbon-carbon double bond are not critical and may be varied in order to achieve a homogeneous solution. Hence, solvents are not required, but inert solvents, such as saturated hydrocarbons, may be used, if desired. In general, larger quantities of catalyst will produce a faster reaction rate. Molar ratios of catalyst to compound having a carbon-carbon double bond between 1 : 1000 and 10 : 1 will normally suffice to accomplish the desired hydroformylation.

Temperatures employed in the process according to the present invention will generally range between 100°C and 300°C and preferably between 125°C and 210°C, a temperature between 150°C and 200°C being generally satisfactory. Temperatures below 100°C and above 300°C may, however, be used.

A particular advantage of the process of the invention resides in the catalyst's stability and its high activity for long periods of time at very low pressures. Consequently, hydroformylation in accordance with the present invention may be

carried out at pressures well below 7 MPa to as low as 0.1 MPa or less. Under comparable conditions, catalysts such as dicobalt octacarbonyl, often decompose and become inactive. The invention is, however, not limited in its applicability to the lower pressures, and pressures in the broad range from 0.1 MPa up to 15 MPa and higher, e.g. up to 35 MPa, may be employed. In general, pressures in the range of from 2 to 10 MPa and particularly in the range of from 3 to 8 MPa are preferred.

The ratio of hydrogen to carbon monoxide charged may vary widely as well. In general, a molar ratio of hydrogen to carbon monoxide of at least 1 : 1 is employed. Very suitable ratios of hydrogen to carbon monoxide comprise those within the range from 1 : 1 to 10 : 1. If conditions are selected that will result primarily in an aldehyde product, only one mole of hydrogen per mole of carbon monoxide enters into reaction with the olefinic compound. When the primary alcohol is the preferred product as in the present invention, two moles of hydrogen and one mole of carbon monoxide react with each mole of olefinic compound.

If desired, the process according to the invention may be carried out in two stages of which the first is carried out to a conversion of 40 to 75% with back-mixing of the reaction mixture and of which the second is carried out to a conversion of at least 95% without back-mixing, as described in British patent specification 1,563,218.

The process according to the invention is applicable to organic compounds having at least one carbon-carbon double bond. Thus, it may be applied to alkenes having from 2 to, for example 20 carbon atoms per molecule, for example alpha-alkenes, such as ethylene, propene, 1-butene, 1-pentene, 1-octene, 1-hexadecene, 1-eicosene and alkenes with a non-terminal double bond, such as 2-butene, 2-hexene, 4-decene, 5-dodecene and 9-eicosene. Other starting alkenes are branched alkenes. Other examples of starting organic compounds are cyclo-alkenes, for example cyclohexene. In the case of polyalkenes, it is possible to hydroformylate only one of the alkenic sites or a part or all of these sites.

The above-mentioned combination of the said complexes with the said salts is believed to be novel. The invention, therefore, also provides a stabilized composition comprising (1) one or more complexes comprising in the molecule an atom of a transition metal having an atomic number of from 22 to 29, of from 40 to 47 or of from 72 to 79, carbon monoxide as a biphyllic ligand and at least one molecule of a biphyllic ligand containing trivalent phosphorus, trivalent arsenic or trivalent antimony, (2) a solvent and (3) one or more dissolved salts of one or more alkyl-substituted arylsulphonic acids. The transition metal is preferably one from Group VIII of the Periodic Table of the Elements. Cobalt is the preferred transition metal in these stabilized compositions. The solvent may be an alcohol or a mixture of alcohols. The stabilized compositions can be stored at atmospheric pressure.

The following Examples further illustrate the invention. The autoclave used in the experiments had a capacity of 1.5 l and was cylindrical, was made of stainless steel (A.I.S.I. 316; 18-20% Cr, 10% Ni, 2.5% Mo, balance Fe), and provided with a mechanical stirrer, gas inlet and sampling cock and surrounded by a jacket through which heating fluid could be circulated.

As biphyllic ligands a mixture of 40 mol.% 9-eicosyl-9-phosphabicyclo[4.2.1]nonane and 60 mol.% 9-eicosyl-9-phosphabicyclo[3.3.1]nonane was used. The $H_2/CO$ mixture used consisted of two volume parts of hydrogen and one volume part of carbon monoxide. The expression "LINEVOL 911" used below is a trade name for a mixture of primary alkanols having 9-11 carbon atoms per molecule, of which at least 80% has a straight carbon chain. The expression "DOBANOL 45" is a trade name for a mixture of n-alkanols having 14-15 carbon atoms per molecule.

The flashing apparatus was a cylindrical glass wiped-film evaporator (Leybold type) surrounded by a jacket through which heating fluid could be circulated and provided with a cooling pipe arranged co-axially with the cylinder; the distillate

and residue were withdrawn at the bottom of the apparatus. The heating fluid in the jacket was kept at a temperature of 200$^{o}$C and the rotor which put the wipers into motion was operated at a speed of 200 revolutions per minute.

The samples were analyzed for "total cobalt" and "active cobalt" by means of infrared analysis. The "total cobalt" is the sum of the cobalt in the cobalt compounds in the dissolved hydroformylation catalyst (herein referred to as "active cobalt") and the cobalt in the dissolved catalytically inactive cobalt compounds (herein referred to as "inactive cobalt"). The active cobalt consists of the cobalt in the complex $HCo(CO)_3$.phosphine (the actual hydroformylation catalyst) and includes the cobalt in the species $Co_2(CO)_6$.(phosphine)$_2$, because on cooling the sample to ambient temperature part of the $HCo(CO)_3$.phosphine complex is converted into $Co_2(CO)_6$.(phosphine)$_2$ and hydrogen. To retard this conversion, the samples as taken from the autoclave were first cooled to ambient temperature and then the pressure was let down.

Comparative Experiment I and Example I

The experiments described below show that even at low hydroformylation pressure the presence of SABS 813 keeps much more active cobalt and inactive cobalt in solution.

The autoclave was charged with:

(1) a mixture (377.5 g) of equal molar portions of 1-octene, 1-nonene and 1-decene (1 mol. of each alkene);

(2) a solution (27.7 g) of the biphyllic ligands (in total 5.21 g or 0.0124 mol.) in a mixture of equal molar portions of 1-octene, 1-nonene and 1-decene;

(3) a solution (8.6 g) of cobalt 2-ethylhexanoate in 2-ethylhexanol (containing 0.0136 mol. Co or 9.35%w Co, calculated as metal on solution) and

(4) a solution (10 ml) of potassium hydroxide in ethanol (6.4%w KOH or 0.009 mol. KOH).

Hereafter, the temperature of the heating fluid in the jacket was kept at 180°C and a $H_2/CO$ mixture having a pressure of 6.0 MPa was admitted above the liquid in the autoclave. After maintaining this pressure for one hour, the pressure was reduced to 1.0 MPa and kept at this value; this pressure is well below the pressure range of 3-9 MPa in which this hydroformylation catalyst is usually applied and was used to speed up the decomposition of the catalyst. Samples were taken after the reaction times stated in Table I and analyzed for total cobalt and active cobalt contents.

Two experiments were carried out as described above, one in the absence of SABS 813 (Comparative Experiment I) and one in the presence of 0.25 mol. of SABS 813 per mol. of cobalt, added to the autoclave contents as a 30%w aqueous solution (Example I). Table I presents the results:

TABLE I

Addition of SABS 813

| Reaction time, h | none (Comparative Experiment I) cobalt content, mmol./l total | active | inactive | 0.25 mol./mol. Co (Example I) cobalt content, mmol./l total | active | inactive |
|---|---|---|---|---|---|---|
| 0.25 | 29.0 | 23.5 | 5.5 | 28.9 | 20.4 | 8.5 |
| 0.50 | 28.6 | 18.5 | 10.1 | 27.7 | 17.4 | 10.3 |
| 1 | 28.8 | 17.0 | 11.8 | 28.3 | 17.2 | 11.1 |
| 1.5 | 21.4 | 14.2 | 7.2 | 21.6 | 14.2 | 7.4 |
| 2 | 20.2 | 13.8 | 6.4 | 20.8 | 14.2 | 6.6 |
| 2.5 | 18.6 | 12.3 | 6.3 | 21.3 | 14.2 | 7.1 |
| 3 | 17.8 | 12.1 | 5.7 | 22.4 | 14.0 | 8.4 |
| 3.5 | 17.8 | 12.1 | 5.7 | 20.5 | 13.7 | 6.8 |
| 4 | 17.0 | 11.2 | 5.8 | 20.9 | 13.7 | 7.2 |
| 4.5 | 16.3 | 10.7 | 5.6 | 20.4 | 13.9 | 6.5 |
| 5 | 16.4 | 10.5 | 5.9 | 20.1 | 12.7 | 7.4 |

At the end of Comparative Experiment I some cobalt was deposited on the inner wall of the autoclave, whilst at the end of Example I no deposited cobalt was observed. At the end of both experiments the yield of alkanols with 9-11 carbon atoms per molecule was 55%.

Comparative Experiment II and Example II

The experiments described below show that after flashing a reaction mixture, obtained by the process according to the present invention the catalyst can be completely regenerated, no cobalt being formed, whilst after flashing a reaction mixture obtained in the same manner but in the absence of SABS 813 only part of the catalyst can be regenerated and cobalt is deposited.

The autoclave was charged with:

(1) "DOBANOL" 45 (500 g);

(2) 9.0 g (0.0213 mol.) of the biphyllic ligands;

(3) a solution (13.5 g) of cobalt 2-ethylhexanoate in 2-ethylhexanol (containing 0.0211 mol. Co or 9.25%w Co, calculated as metal on solution) and

(4) a solution (10 ml) of potassium hydroxide in ethanol, containing 1.2 g (0.021 mol.) KOH.

Hereafter, the temperature of the heating fluid in the jacket was kept at 180°C and a $H_2/CO$ mixture having a pressure of 4.5 MPa was admitted above the liquid in the autoclave. This pressure was maintained for 90 minutes, to ensure that the contents of the autoclave had reached equilibrium.

After a period of 90 minutes reckoned from the start the liquid was drained out of the autoclave via a cooling coil under the reactor and charged into the feed vessel of the flashing apparatus. From there the liquid was fed into the flashing apparatus at a rate of 0.5 ml/s. The pressure in the flashing apparatus was 6 kPa. In 20 minutes the total charge had passed through the apparatus and the residue, containing all the catalyst species, had resided for an average of 10 minutes in the receiving vessel, where the temperature was

about 50°C and the pressure 6 kPa. Then, the residue and distillate were mixed and introduced into the autoclave; at this moment 130 minutes had elapsed after the start of the experiment. Now heating of the autoclave was resumed and a $H_2$/CO mixture having a pressure of 4.5 MPa was admitted above the liquid in the autoclave for 105 minutes to regenerate the catalyst.

Samples were taken from the contents of the autoclave at the times stated in Table II and analyzed for total cobalt and active cobalt contents.

Two experiments were carried out as described above, one in the absence of SABS 813 (Comparative Experiment II) and one in the presence of 0.30 mol. of SABS 813 per mol. of cobalt, added in 30%w solution (8.5 g) in "LINEVOL" 911.

Table II presents the results:

TABLE II

| Time, min. | Addition of SABS 813: none (Comparative Exp. II) cobalt content, mmol./l total | active | inactive | 0.30 mol./mol. Co (Example II) cobalt content, mmol./l total | active | inactive |
|---|---|---|---|---|---|---|
| 60 | 38.1 | 22.4 | 15.7 | 39.8 | 22.0 | 17.8 |
| 90 | 40.3 | 23.3 | 17.0 | 39.7 | 22.1 | 17.6 |
| 240 | 32.8 | 20.0 | 12.8 | 39.7 | 22.1 | 17.6 |

In Comparative Experiment II the parts of the flashing apparatus that had come into contact with liquid containing dissolved cobalt compounds were covered with a layer of cobalt, whilst in Example II these parts were free from cobalt.

Comparative Experiment III and Example III

In the experiments described below hydroformylation was avoided by starting from alkanols. These experiments were carried out as hydroformylation consumes carbon monoxide

and the concentration thereof in the liquid might well drop far below that corresponding with the carbon monoxide partial pressure measured in the gas phase above the reaction mixture. The results show that in this case, too, at pressures usually applied in hydroformylation the presence of SABS 813 keeps much more active cobalt and inactive cobalt in solution.

The autoclave was charged with:

(1) "LINEVOL" 911 (500 g);

(2) the biphyllic ligands (9.0 g or 0.0213 mol.);

(3) a solution (19.5 g) of cobalt 2-ethylhexanoate in 2-ethylhexanol (containing 0.0211 mol. Co or 6.4%w Co, calculated as metal on solution) and

(4) a solution of potassium hydroxide (1.22 g or 0.0218 mol.) in ethanol (14 g).

Hereafter, the temperature of the heating fluid in the jacket was kept at 197°C and a $H_2/CO$ mixture having a pressure of 4.5 MPa was admitted above the liquid in the autoclave. After one hour this pressure was reduced to 3.0 MPa and kept at this value. Samples were taken after the reaction times stated in Table III and analyzed for total cobalt and active cobalt contents.

Two experiments were carried out as described above, one in the absence of SABS 813 (Comparative Experiment III) and one in the presence of 0.30 mol. of SABS 813 per mol. of cobalt, added to the autoclave as a 25%w solution (1.7 g) in "LINEVOL" 911.

Table III presents the results:

TABLE III

| Time, h | Addition of SABS 813: none (Comparative Exp. III) cobalt content, mmol./l total | active | inactive | 0.30 mol./mol. Co (Example III) cobalt content, mmol./l total | active | inactive |
|---|---|---|---|---|---|---|
| 0.5 | 42.1 | 23.8 | 18.3 | 41.3 | 22.8 | 18.5 |
| 1 | 39.3 | 21.6 | 17.7 | 40.9 | 22.6 | 18.3 |
| 1.5 | 34.6 | 20.1 | 14.5 | 39.2 | 21.8 | 17.4 |
| 2 | 33.0 | 18.5 | 14.5 | 38.4 | 21.3 | 17.1 |
| 3 | 29.4 | 16.6 | 12.8 | 38.4 | 21.3 | 17.1 |
| 4 | 25.9 | 13.8 | 12.1 | 35.7 | 19.5 | 16.2 |
| 5 | 24.1 | 12.7 | 11.4 | 35.7 | 19.3 | 16.4 |
| 6 | 22.1 | 11.4 | 10.7 | 35.1 | 18.9 | 16.2 |

Comparative Experiment IV and Example IV

These experiments show that the presence of SABS 813 considerably enhances the proportions of active and inactive cobalt that remain dissolved during storage of a catalyst solution.

The two liquids obtained in Comparative Experiment III and Example III were stored at 20°C and atmospheric pressure (Comparative Experiment IV and Example IV, respectively). Samples were taken after the periods stated in Table IV and analyzed for total cobalt and active cobalt contents.

Table IV presents the results:

-18-

TABLE IV

| Time, days | Presence of SABS 813: none (Comparative Exp. IV) cobalt content, mmol./l total | active | inactive | 0.30 mol./mol. Co (Example IV) cobalt content, mmol./l total | active | inactive |
|---|---|---|---|---|---|---|
| 0 | 22.1 | 11.4 | 10.7 | 35.1 | 18.9 | 16.2 |
| 2 | 12 | 8 | 4 | 25 | 13 | 12 |
| 6 | 11 | 7 | 4 | 24 | 11 | 13 |
| 12 | 8 | 5 | 3 | 21 | 9.5 | 11.5 |
| 17 | 7 | 4.5 | 2.5 | 21 | 9.5 | 11.5 |
| 22 | 7 | 4.5 | 2.5 | 21 | 9.5 | 11.5 |

Comparative Experiment V and Example V

The experiments described below show that even at an extremely low pressure the presence of SABS 913 considerably reduces the deposition of cobalt.

A mixture of 1-tridecene and 1-tetradecene was hydroformylated in a continuously operated reactor in the presence of a catalyst prepared by combining the biphyllic ligands, cobalt 2-ethylhexanoate and a solution of potassium hydroxide in ethanol. A slip stream of the reaction mixture thus obtained was passed from the reactor to the flashing apparatus at a rate of 4 ml/min. The pressure in the flashing apparatus had a value of 27 Pa. The compositions of the feed, distillate and residue are given in Table V.

Ten minutes after the flashing apparatus was started the walls that had come into contact with liquid containing cobalt compounds were completely covered with a black deposit of cobalt and did not allow any light to pass (Comparative Experiment V).

TABLE V

| | Amount, % on feed | Composition, %w *) | | |
|---|---|---|---|---|
| | | n-tetradecanol and n-pentadecanol | compounds boiling above 285°C | hydrocarbons |
| Feed to flashing apparatus | 100 | 30.1 | 69.8 | 0.1 |
| Distillate | 70 | 37.5 | 62.4 | 0.1 |
| Residue | 30 | 1.1 | 98.8 | 0.1 |

*) Calculated on the sum of the three figures.

The flashing apparatus used for Comparative Experiment V was cleaned with aqueous nitric acid and an experiment was carried out as described for Comparative Experiment V, but this time SABS 913 (30%w of SABS 913 in water and using 0.25 mol. of SABS 913 per mol. of cobalt) was added to the reactor contents (Example V).

Thirty minutes after the flashing was started the walls that had come into contact with liquid containing cobalt compounds were covered with considerably less cobalt than the walls inspected in Comparative Experiment V after 10 minutes' flashing.

The compositions of the feed to the flashing apparatus and of the top and bottom products were the same as in Comparative Experiment V (see Table V).

Comparative Experiment VI and Examples VI and VII

An equimolar mixture of 1-undecene and 1-dodecene was hydroformylated and the reaction product obtained was flashed, all as described in Example V. A sample of the residue withdrawn from the flashing apparatus was introduced into a flask provided with a reflux condenser and stirrer. A clear and bright test coupon of mild steel was immersed in the sample. The contents of the flask were kept under nitrogen and heated under reflux (180°C). Three experiments were thus carried out:

Comparative Experiment VI in the absence of SABS 913 and Examples VI and VII in the presence of different amounts thereof, added in a 30%w solution in water. After 72 hours' refluxing the appearance of the three test coupons was examined. Table VI presents the results:

TABLE VI

| | SABS 913 mol./mol. Co | Appearance of test coupon |
|---|---|---|
| Comparative Exp. VI | 0 | black from cobalt deposition |
| Example VI | 0.01 | dark brown from cobalt deposition |
| Example VII | 0.1 | unchanged, no cobalt deposition |

# CLAIMS

1. A process for the preparation of alcohols and/or aldehydes, characterized in that a compound having a carbon-carbon double bond is reacted with carbon monoxide and hydrogen in the presence of (1) one or more complexes comprising in the molecule an atom of a transition metal having an atomic number of from 22 to 29, of from 40 to 47 or of from 72 to 79, carbon monoxide as a biphyllic ligand and at least one molecule of a biphyllic ligand containing trivalent phosphorus, trivalent arsenic or trivalent antimony, and (2) one or more dissolved salts of one or more alkyl-substituted arylsulphonic acids.

2. A process as claimed in claim 1, characterized in that the salts of the alkyl-substituted arylsulphonic acids are salts of alkyl-substituted benzenesulphonic acids.

3. A process as claimed in claim 1 or 2, characterized in that the alkyl group or alkyl groups in the salts of the alkyl-substituted arylsulphonic acids have in the range of from 6 to 30 carbon atoms.

4. A process as claimed in any one of the preceding claims, characterized in that the alkyl group or alkyl groups in the salts are unbranched and are for the greater part bound with a tertiary carbon atom to the aromatic nucleus.

5. A process as claimed in claim 4, characterized in that the salts of the alkyl-substituted arylsulphonic acids are alkali metal, alkaline earth metal or ammonium salts.

6. A process as claimed in claims 4 and 5, characterized in that a mixture of sodium salts of p-alkylbenzenesulphonic acids is used, in which mixture the alkyl groups have in the range of from 8 to 13 carbon atoms.

7. A process as claimed in any one of the preceding claims, characterized in that the molar ratio of salt of alkyl-substituted arylsulphonic acid to transition metal is in the range of from 0.005 to 2.

8. A process as claimed in any one of the preceding claims, characterized in that the biphyllic ligand containing trivalent phosphorus is a bicyclic heterocyclic tertiary phosphine.

9. A process as claimed in claim 8, characterized in that the phosphine is 9-eicosyl-9-phosphabicyclo [4.2.1] nonane or 9-eicosyl-9-phosphabicyclo [3.3.1] nonane.

10. A process as claimed in any one of the preceding claims, characterized in that the transition metal is one from Group VIII of the Periodic Table of the Elements.

11. A process as claimed in claim 10, characterized in that the transition metal is cobalt.

12. A process as claimed in any one of the preceding claims, characterized in that it is carried out at a pressure in the range of from 2 to 10 MPa.

13. A process as claimed in any one of the preceding claims, characterized in that it is carried out in an alkaline medium.

14. A process as claimed in claim 13, characterized in that the alkaline medium contains potassium hydroxide.

15. A process as claimed in claim 1, substantially as hereinbefore described with reference to Examples I, V and VI.

16. Alcohols or aldehydes whenever prepared by a process as claimed in any one of the preceding claims.

17. A stabilized composition comprising (1) one or more complexes comprising in the molecule an atom of a transition metal having an atomic number of from 22 to 29, of from 40 to 47 or of from 72 to 79, carbon monoxide as a biphyllic ligand and at least one molecule of a biphyllic ligand containing trivalent phosphorus, trivalent arsenic or trivalent antimony, (2) a solvent and (3) one or more dissolved salts of one or more alkyl-substituted arylsulphonic acids.

18. A stabilized composition as claimed in claim 17, characterized in that the transition metal is one from Group VIII of the Periodic Table of the Elements.

19. A stabilized composition as claimed in claim 18, characterized in that the transition metal is cobalt.

20. Stabilized compositions as claimed in claim 17, substantially as hereinbefore described with reference to Examples II, III and IV.

European Patent Office

# EUROPEAN SEARCH REPORT

0024761
Application number
EP 80200774.0

| Category | DOCUMENTS CONSIDERED TO BE RELEVANT<br>Citation of document with indication, where appropriate, of relevant passages | Relevant to claim | CLASSIFICATION OF THE APPLICATION (Int. Cl. 3) |
|---|---|---|---|
| | US - A - 3 976 703 (WILKES)<br>+ Totality +<br>-- | 1-7,10, 11 | C 07 C 27/22<br>C 07 C 45/50<br>C 07 C 29/16<br>B 01 J 31/20<br>B 01 J 31/24 |
| | US - A - 3 420 898 (WINKLE)<br>+ Examples 3-7; claims +<br>-- | 1,8-11 | |
| D | GB - A - 1 254 063 (SHELL)<br>+ Example 3; claims +<br>---- | 1,8,10, 11,13, 14 | |
| | | | TECHNICAL FIELDS SEARCHED (Int.Cl. 3)<br>C 07 C 27/00<br>C 07 C 29/00<br>C 07 C 45/00<br>B 01 J 31/00 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| VIENNA | 13-11-1980 | TENGLER |

EPO Form 1503.1 06.78